(19) Europäisches Patentamt
European Patent Office
Office européen des brevets

(11) Numéro de publication: **0 061 397**
**B1**

# (12) FASCICULE DE BREVET EUROPÉEN

(45) Date de publication du fascicule du brevet: **15.01.86**

(21) Numéro de dépôt: **82400495.6**

(22) Date de dépôt: **18.03.82**

(51) Int. Cl.⁴: **C 07 D 401/12**, C 07 D 405/12, C 07 D 409/12, C 07 D 239/48, A 61 K 31/505 // (C07D401/12, 239:48, 213:34), (C07D405/12, 239:48, 307:38, 317:56), (C07D409/12, 239:48, 333:18)

(54) **Nouveaux dérivés solubles N2 substitués de la diamino-2,4-benzyl-5-pyrimidines, leur procédé de préparation et médicaments les contenant.**

(30) Priorité: **20.03.81 FR 8105592**

(43) Date de publication de la demande: **29.09.82 Bulletin 82/39**

(45) Mention de la délivrance du brevet: **15.01.86 Bulletin 86/3**

(84) Etats contractants désignés: **AT BE CH DE GB IT LI LU NL SE**

(56) Documents cité:
**FR-A-2 358 148**
**FR-A-2 397 407**

**Le dossier contient des informations techniques présentées postérieurement au dépôt de la demande et ne figurant pas dans le présent fascicule.**

(73) Titulaire: **S.A. PANMEDICA Société dite:, Zone Industrielle - Ilot J, F-06510 Carros (FR)**

(72) Inventeur: **Laruelle, Claude, Avenue Bellevue, F-06270 Villeneuve Loubet (FR)**
Inventeur: **Lepant, Marcel, L'Escoundu, Allée Clairefontaine, F-06140 Vence (FR)**

(74) Mandataire: **Ores, Irène, CABINET ORES 6, Avenue de Messine, F-75008 Paris (FR)**

**EP 0 061 397 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention est relative à des nouveaux dérivés solubles $N_2$ substitués de la diamino-2,4-benzyl-5 pyrimidine, à leur procédé de préparation et aux médicaments les contenant. On connaît depuis longtemps (Brevet américain 3 049 544 de 1962) la diamino-2,4,(3,4,5-triméthoxybenzyl)-5 pyrimidine en tant qu'agent antibactérien à trés large spectre englobant aussi bien les cocci Gram-positifs que les cocci Gram-négatifs et les bacilles Gram-négatifs. Son mécanisme d'action se rapporoche de celui des sulfamides, il interfère avec la déhydrofolateréductase, enzyme qui intervient dans réduction de l'acide folique, métabolite nécessaire à la thymine. Il agit dans une étape plus tardive que les sulfamides pour inhiber la synthèse de l'acide folique. Ceci explique son action synergique avec les sulfamides.

Malheureusement, ce produit est très peu soluble dans l'eau et il est très difficile de préparer par exemple des solutions injectables. On a recours pour y parvenir, soit à des suspensions (par exemple Brevet français 2 085 703) soit à des solvants organiques. Un très grand progrès dans ce sens a été effectué grâce aux travaux de MM. Rombi et Dick (brevets français 2 358 148 et 2 397 407. Les produits synthétisés obtenus, à savoir les dérivés $N_2N_4$ substitués de formule I.

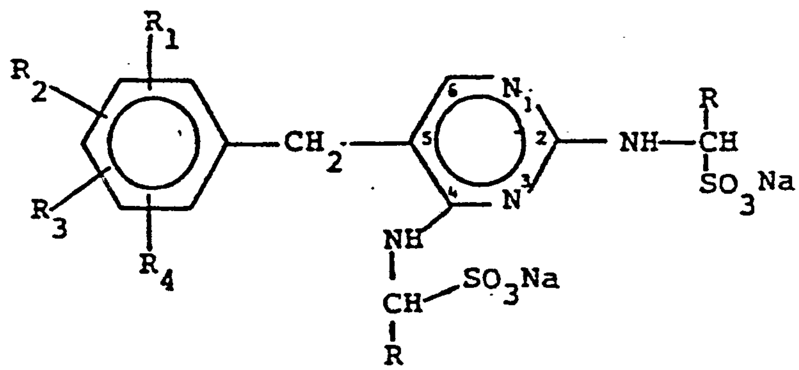

(I)

où les dérivés $N_4$ substitués de formule II:

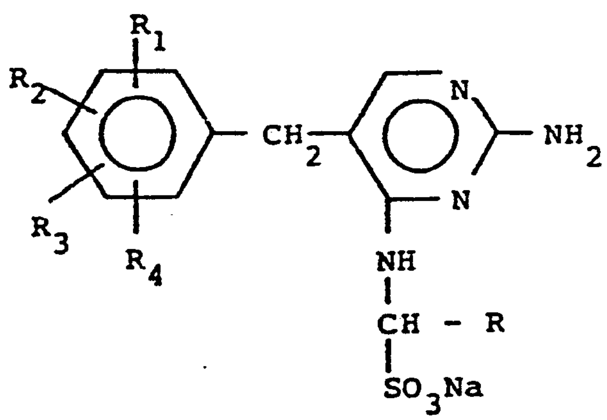

(II)

quoique très solubles dans l'eau, présentent toutefois encore deux sortes d'inconvénients:

-les produits obtenus ne sont jamais chimiquement purs, mais constituent un mélange variable des dérivés disubstitués $N_2N_4$ et monosubstitués en $N_4$,

-leur stabilité aux pH acides laisse à désirer et ils sont donc difficilement purifiables.

La présente invention a pour objet de nouveaux dérivés solubles $N_2$ substitués de la diamino-2,4-benzyl-5 pyrimidine répondant à la formule III ci-après:

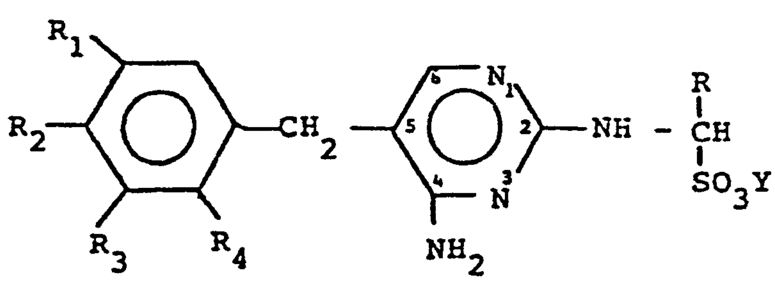

(III)

dans laquelle:

$R_1$, $R_2$, $R_3$, $R_4$ qui peuvent être identiques ou différents représentent un atome d'hydrogène, un atome d'halogène ou un groupe alkyle, thioalkyle, alcoxy- ou benzyloxy- ou alkyloxyalcoxy-, Y représente un

atome d'hydrogène, un métal alcalin, ou une base organique pharmaceutiquement compatible,et R repésente un atome d'hydrogène ou un radical alkyle linéaire ou ramifié comprenant de 1 à 7 atomes de carbone, un radical cycloalkyle comprenant de 5 à 8 atomes de carbone, un radical phényl éventuellement substitué par des substituants tels que, halogène, nitro-, hydroxyle, dialkylamino-, alcoxy-, alkyl de $C_1$ à $C_3$, deux substituants adjacents pouvant constituer un cycle alkyldioxy-, le furanne, le thiophene, la pyridine.

Parmi les dérivés des diamino-2,4 pyrimidine, on peut citer, de préférence, la (triméthoxy-3,4,5 benzyl)-5 diamino-2,4 pyrimidine ou triméthoprime, la (diméthoxy-3,4 benzyl)-5 diamino-2,4 pyrimidine ou diavéridine, la (méthyl-2-diméthoxy-4,5 benzyl)-5 diamino-2,4 pyrimidine ou orméthoprime.

Outre la parfaite stabilite et la pureté analytique des produits obtenus, la Demanderesse a également constate de manière surprenante, que l'activité des produits conformes à la présente invention est de beaucoup supérieure à celle des produits $N_2N_4$ ou $N_4$ substitués.

La présente invention a également pour objet un procédé de préparation de ces produits de formule III conformes à l'invention, caractérisé en ce que l'on fait réagir dans un solvant approprié tel que la pyridine, notamment, un dérivé de diamino-2,4 benzyl-5 pyrimidine avec un aldéhyde et l'anhydride sulfureux, puis en ce qu'on isole le produit de la réaction.

Suivant un mode de réalisation avantageux du procédé objet de la présente invention, les quantités de diamino-2,4 benzyl-5 pyrimidine et d'aldéhyde, sont stoechiométriques, tandis que l'anhydride sulfureux est utilisé en excés.

Suivant un autre mode de réalisation avantageux du procédé objet de la présente invention, le produit de la réaction est isolé par dilution du milieu réactionnel par un solvant miscible à la pyridine tel que l'éther ou un hydrocarbure.

Suivant une modalité particulière de ce mode de réalisation, le produit isolé est en outre purifié par lavage à l'alcool aqueux.

La présente invention a en outre pour objet des médicaments solubles dans l'eau contenant au moins un dérivé conforme à la présente invention.

En effet, ces dérivés sont très solubles dans l'eau et permettent d'obtenir des solutions aqueuses contenant jusqu'à 50 % en poids/volume. Les produits obtenus sont des poudres microcristallines blanches, stables sous leurs formes acides ou salifiées. Les solutions aqueuses des sels se présentent sous la forme d'un liquide transparent incolore et de pH voisin de la neutralité.

Outre les dispositions qui précèdent, l'invention comprend encore d'autres dispositions qui ressortiront de la description qui va suivre.

L'invention vise particulièrement de nouveaux dérivés $N_2$ substitués de la diamino-2,4 benzyl-5 pyrimidine remarquablement stables et très solubles dans l'eau, qui conviennent parfaitement pour la fabrication de médicaments injectables, ainsi que les moyens propres à leur réalisation et à la mise en oeuvre des procédés de fabrication de ces nouveaux dérivés.

L'invention pourra être mieux comprise à l'aide du complément de description qui va suivre, qui se réfère à des exemples de fabrication, à l'analyse des produits obtenus, ainsi qu'à un compte-rendu d'expérimentations pharmacologiques portant sur les médicaments conformes à la presente invention.

Il doit être bien entendu toutefois que ces exemples, les différents résultats analytiques et ce compte-rendu d'expérimentations pharmacologiques, sont donnés uniquement à titre d'illustration de l'objet de l'invention.

Dans les exemples qui vont suivre, les chromatographies en couches minces (CCM) ont été réalisées sur plaque KIESELGEL F 254 avec révélation en lumière UV à 254 nm dans les systèmes de migration suivants:

- Système A: chloroforme: 80, méthanol:20, acide acétique: 20 - eau: 1
- Système B: chloroforme: 80 - éthanol: 40 acide formique: 5

La triméthoprime a un Rf de 0,6 dans le systéme A et 0,3 dans le systéme B.

## EXEMPLE 1: ACIDE $N_2$ METHANE SULFONIQUE DE LA DIAMINO-2,4 (TRIMETHOXY-3,4,5 BENZYL)-5 PYRIMIDINE ET SON SEL DE SODIUM

$$\left[(III)\quad \begin{array}{l} R = H \\ Y = H,\ Na \end{array}\right].$$

On ajoute successivement 2,22 g (74 mM) de paraformaldéhyde et 19,6 g (70 mM) de triméthoprime à 80 ml de pyridine et on introduit 70 g d'anhydride sulfureux en deux heures, la température se maintient spontanément à 45-50°. On porte ensuite à 80°C pendant 8 heures. Aprés refroidissement, on coule dans 500 ml d'éther et on sépare le précipité. Le précipité est mis en suspension dans l'eau et redissous par addition de soude diluée en quantité suffisante pour pH = 9,70. Après filtration d'un léger insoluble, on reprécipite par ajout d'acide chlorhydrique

dilué quantité suffisante pour pH = 1/2. Le précipité est filtré, lavé abondamment à l'eau puis à l'éthanol. Aprés séchage, on obtient la forme acide du dérivé recherché à l'état pur sous forme de cristaux blancs de pF 176°C. La CCM donne un Rf de 0,33 dans le système A et 0,16 dans le système B. Cette forme acide peut être traitée en milieu aqueux par de la soude diluée jusqu'à pH 9,50 pour donner le sel sodique qu'on isole par évaporation et séchage [pF supérieur à 250°C (dec)] [RMN:2 H à 4,8 ppm (m)].

**EXEMPLE 2: ACIDE $N_2$ ETHANE SULFONIQUE DE LA DIAMINO-2,4 (TRIMETHOXY-3,4,5 BENZYL)-5 PYRIMIDINE**

[(III) R = $CH_3$
Y = H, Na]

On ajoute successivement 14,5 g (0,05 mole) de triméthoprime et 2,2 g (0,05 mole) d'aldéhyde acétique à 100 ml de pyridine. On introduit à raison d'environ 10 g par heure, 40 g d'anhydride sulfureux, puis on porte à 60°C pendant 8 heures. Après refroidissement, on coule sur 500 ml d'éther, on filtre le précipité et on sèche à l'air. Le produit brut est remis en suspension dans l'eau et traité par de la soude diluée jusqu'à pH 8,80. On filtre un léger insoluble et le filtrat est ensuite acidifié par de l'acide chlorhydrique dilué jusqu'à pH 2,50. Le précipité est filtré, lavé soigneusement à l'eau, puis à l'éthanol. Aprés séchage, on obtient le dérivé recherché sous forme de beaux cristaux blancs de pF 192°C. La CCM donne un spot de Rf de 0,43 dans le système A et de 0,31 dans le système B. La forme acide peut être convertie en son sel de sodium par traitement de la suspension aqueuse du produit précédant par de la soude diluée jusqu'à pH 8,80. Après évaporation et séchage, on obtient le sel de sodium du dérivé recherché de pF 178/180°C (RMN: $CH_3$ (d) 3 H à 1,3 ppm, CH (m) 1 H à 4,7 ppm).

**EXEMPLE 3: ACIDE $N_2$ BUTANE SULFONIQUE DE LA DIAMINO-2,4 (TRIMETHOXY-3,4,5 BENZYL)-5 PYRIMIDINE**

[(III) : R = $-CH_2-CH_2-CH_3$
Y = H, Na]

On introduit successivement 14,5 g (0,050 mole) de triméthoprime et 3,6 g (0,050 mole) de butyraldéhyde dans 200 ml de pyridine et on introduit lentement sous agitation, environ 45 g d'anhydride sulfureux. Les réactifs passent rapidement en solution, tandis que la température intérieure monte spontanément à 35-40°C pour se stabiliser. On abandonne 24 heures à température ordinaire, puis on coule sur un litre d'éther. Le précipité est filtré, lavé à l'éther et séché à l'air. Le produit brut est ensuite mis en suspension aqueuse et traité par de la soude diluée jusqu'à pH 8,50. On filtre un léger insoluble puis on acidifie par de l'acide chlorhydrique dilué, jusqu'à pH 2,0; la forme acide précipite à nouveau en beaux cristaux blancs qu'on filtre et lave abondamment à l'eau, puis à l'éthanol. Après séchage, on obtient la forme acide du dérivé recherché de pF 165°C. La CCM donne un Rf de 0,50 dans le systéme A et de 0,40 dans le systéme B. On peut remettre la forme acide en suspension aqueuse et la traiter par de la soude diluée jusqu'à pH 8,50. Après évaporation et séchage, on obtient le sel de sodium du dérivé recherché de pF 180°C (RMN: $CH_3-(CH_2)_2$ (m) 7 H à 1,3 ppm, CH (m) 1 H à 4,7 ppm).

**EXEMPLE 4: ACIDE $N_2$ (METHYL-2) PROPANE SULFONIQUE DE LA DIAMINO-2,4 (TRIMETHOXY-3,4,5 BENZYL)-5 PYRIMIDINE ET SON SEL DE SODIUM**

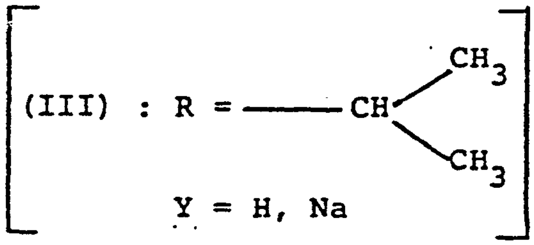
(III) : R =
Y = H, Na

On ajoute 14,5 g (0,05 mole) de triméthoprime et 3,6 g (0,05 mole) d'aldéhyde isobutyrique à 100 ml de pyridine, puis on introduit sous agitation, environ 45 g d'anhydride sulfureux, la température se stabilise vers 40°C. Aprés quelques heures à température ordinaire, la solution est coulée dans un grand volume d'éther. Aprés isolement du brut, on traite comme précédemment en dissolvant à pH 9,0, on élimine quelques impuretés par filtration et on reprécipite par de l'acide chlorhydrique dilué. La forme acide est alors filtrée, lavée à l'eau trés soigneusement, puis séchée (pF = 182°C).

La CCM donne un seul spot de Rf 0,58 dans le systéme A et de 0,45 dans le système B. On peut obtenir le sel de sodium par neutralisation de la suspension aqueuse de la forme acide par de la soude diluée jusqu'à pH 9,20. La solution, après évaporation et séchage, donne la forme salifiée de pF 160°C. (RMN: $CH_3$ (d) 6 H à 1 ppm, CH (m) 1 H à 2,2 ppm, CH (m) 1 H à 4,8 ppm).

**EXEMPLE 5: ACIDE $N_2$ (DIMETHYL-2,2) PROPANE SULFONIQUE DE LA DIAMINO-2,4 (TRIMETHOXY-3,4,5 BENZYL)-5 PYRIMIDINE**

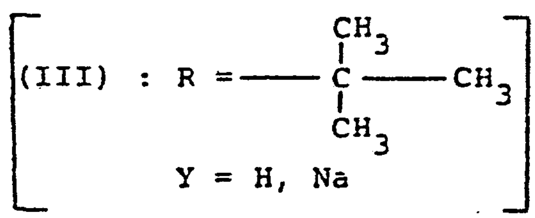
(III) : R =
Y = H, Na

On ajoute successivement 14,5 g (0,050 mole) de triméthoprime et 4,5 g (0,050 mole) d'aldéhyde pivalique à 100 ml de pyridine. Aprés traitement selon la technique habituelle, on remet le produit brut en suspension dans 300 ml d'eau et on alcalinise par de la soude diluée jusqu'â pH 9,0. On filtre un léger insoluble, puis on acidifie par de l'acide chlorhydrique dilué jusqu'à pH 2,50. Le précipité apparu est filtré, lavé abondamment à l'eau, puis à l'éthanol. Aprés séchage, on obtient la forme acide du dérivé recherché de pF 170°C. La CCM ne donne qu'un seul spot de Rf 0,70 dans le système A et de 0,55 dans le système B. On peut remettre ce produit en suspension dans l'eau et par traitement par de la soude diluée jusqu'à pH 8,70; on obtient, aprés évaporation et séchage, la forme sodique du dérivé recherché de pF = 155°C (RMN: $CH_3$ (s) 9 H à 1 ppm CH (m) 4,8 ppm).

**EXEMPLE 6: ACIDE $N_2$ (DIMETHYL-2,3) PENTANE SULFONIQUE DE LA DIAMINO-2,4 (TRIMETHOXY-3,4,5 BENZYL)-5 PYRIMIDINE ET SON SEL DE SODIUM**

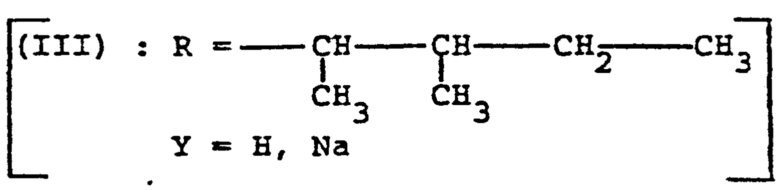
(III) : R =
Y = H, Na

On ajoute à température ordinaire, 14,5 g (0,05 mole) de triméthoprime et 5,6 g (0,05 mole) d'aldéhyde 2,3 diméthylvalérique à 100 ml de pyridine anhydre, puis on introduit lentement sous agitation, environ 45 g d'anhydride sulfureux. Les réactifs passent assez rapidement en solution, tandis que la température intérieure se stabilise entre 35 et 40°. On laisse ensuite 24 heures à température ordinaire, puis on coule lentement sur un litre d'éther. Le précipité blanc qui apparaît immédiatement est filtré, lavé à l'éther puis séché. Le produit est remis en suspension aqueuse et dissous par ajout d'un minimum de soude dilué pour atteindre pH 8,0; on filtre un léger insoluble, puis on acidifie par de l'acide chlorhydrique dilué, la forme acide précipite à nouveau sous forme d'une belle cristallisation blanche. On filtre, on lave soigneusement à l'eau et on séche (pF = 182°). La CCM sur Kieselgel donne un seul spot de Rf 0,54 dans le système A et une seule tache également de Rf 0,39 dans le système B. On peut obtenir le sel de sodium de ce dérivé par neutralisation de la suspension aqueuse de la forme acide, par de la soude diluée à pH 8-8,20. La solution limpide est ensuite évaporée et séchée sous vide (RMN: $CH_2$ - $CH_3$ (m) 1 ppm.-CH-(m) 2 H à 2,3 ppm, CH (m) 1 H à 4,8 ppm).

**EXEMPLE 7: ACIDE $N_2$ (ETHYL-2) BUTANE SULFONIQUE DE LA DIAMINO-2,4 (TRIMETHOXY-3,4,5 BENZYL)-5 PYRIMIDINE ET SON SEL DE SODIUM**

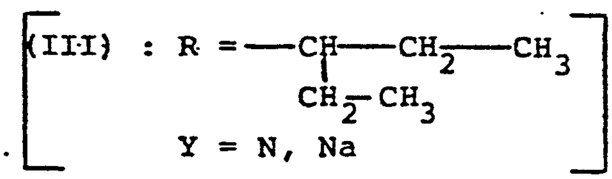

On ajoute 14,5 g (0,05 mole) de triméthoprime et 5,0 g (0,05 mole) d'aldéhyde éthyl-2 butyrique à 100 ml de pyridine, puis on opère comme dans les exemples précédents. Le produit brut séparé de l'éther et séché est repris dans l'eau et mis en solution par ajout de soude diluée jusqu'à pH 9,30. On filtre quelques impuretés, puis on reprécipite la forme acide par addition d'acide chlorhydrique dilué au filtrat jusqu'à pH 2,20. La cristallisation de la forme acide est complétée en refroidissant la suspension à 0°C pendant quelques heures. Par filtration et séchage, on obtient la forme acide du dérivé. La CCM du dérivé donne un seul spot de Rf 0,61 dans le système A et de 0,49 dans le système B. Son point de fusion est 170°C. On peut obtenir la forme alcaline en dispersant le dérivé acide dans l'eau et en le dissolvant par addition du minimum de soude diluée jusqu'à pH 8,90-9,20. Aprés évaporation à basse température de la solution alcaline et sèchage, on obtient le dérivé recherché (RMN: $CH_3$-$CH_2$- (m) 10 H à 1,1 ppm, CH (m) 1 H à 2,2 ppm, CH (m) 1 H à 4,8 ppm).

**EXEMPLE 8: ACIDE $N_2$ PHENYL METHANE SULFONIQUE DE LA DIAMINO2,4 (TRIMETHOXY-3,4,5 BENZYL)-5 PYRIMIDINE ET SON SEL DE SODIUM**

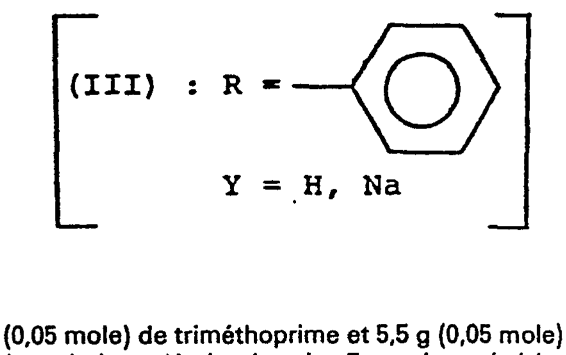

On met en suspension 14,5 g (0,05 mole) de triméthoprime et 5,5 g (0,05 mole) de benzaldéhyde dans 100 ml de pyridine, puis on opère selon la technique décrite dans les Exemples précédents. Le produit brut est mis en suspension dans environ 300 ml d'eau et additionné de soude diluée jusqu'à pH 8,0-9,0. Après filtration d'un léger insoluble, on reprécipite la forme acide par addition d'acide chlorhydrique dilué en quantité suffisante pour pH 2,5. La forme acide est filtrée, lavée, séchée. La CCM du dérivé acide donne un seul spot de Rf = 0,53 dans le système A et de Rf = 0,43 dans le système B et de pF = 166°C. On obtient la forme alcaline en traitant la suspension aqueuse de la forme acide par la quantité minimum de soude diluée pour obtenir la dissolution complète, soit pH 8,40-8,50, puis en évaporant à sec cette solution on obtient le sel de sodium du dérivé recherché à l'état pur. (RMN: arom (s) 5 H à 7,2 ppm, CH (m) à 4,8 ppm).

**EXEMPLE 9: ACIDE $N_2$ (CHLORO-2 PHENYL)METHANE SULFONIQUE DE LA DIAMINO-2,4 (TRIMETHOXY-3,4,5 BENZYL)-5 PYRIMIDINE ET SON SEL DE SODIUM**

(III) : R = 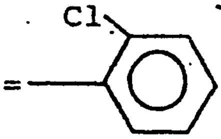

Y = H, Na

On ajoute successivement 14,5 g (0,05 mole) de triméthoprime et 7,0 g (0,05 mole) de chloro-2 benzaldéhyde à 100 ml de pyridine, puis on opère selon la technique decrite dans les exemples précédents. Le produit brut est mis en suspension dans environ 300 ml d'eau, puis traité par de la soude 2 N jusqu'à pH 8,7-8,9. On filtre un léger insoluble et on reprécipite la forme acide par addition d'acide chlorhydrique en quantité suffisante pour pH 2,50.

On maintient à 0°C pendant 24 heures pour terminer la cristallisation, puis on filtre, on lave à l'eau, éventuellement à l'alcool. Aprés séchage, on obtient la forme acide pure. La CCM de la forme acide donne un seul spot de Rf 0,58 dans le systéme A et de 0,54 dans le systéme B. Cette forme acide peut être traitée en milieu aqueux par de la soude diluée jusqu'à pH 8,10-8,20 pour donner la forme sodique du produit qu'on isole par évaporation et séchage. pF = 165°C (RMN: arom (4H) (m) à 7,2 ppm - H (m) à 4,8 ppm).

**EXEMPLE 10: ACIDE $N_2$ (CHLORO-3 PHENYL) METHANE SULFONIQUE DE LA DIAMINO-2,4 (TRIMETHOXY-3,4,5 BENZYL)-5 PYRIMIDINE ET SON SEL DE SODIUM**

(III) : R = 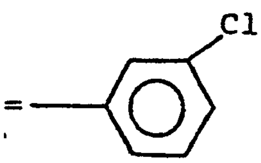

Y = H, Na

On ajoute successivement 14,5 g (0,05 mole) de triméthoprime et 7,0 g (0,05 mole) de chloro-3 benzaldéhyde à 100 ml de pyridine, puis on traite selon les exemples précédents. Le produit brut est mis en suspension dans 350 ml d'eau et additionné de soude 2 N jusqu'à pH 8,10-8,60. On filtre un léger insoluble, puis on acidifie par de l'acide chlorhydrique dilué jusqu'à pH 2,25. La forme acide précipitée est filtrée, lavée à l'eau, puis à l'éthanol absolu. Après séchage, on obtient le dérivé recherché sous forme acide à l'état pur. La CCM sur Kieselgel donne un seul spot de Rf 0,59 dans le système A et de 0,57 dans le système B. Par alcalinisation avec de la soude diluée de la suspension aqueuse de la forme acide à concurrence de pH 8,80-8,70 on obtient le sel de sodium du dérivé recherché après évaporation et séchage. pF = 176°C (RMN; arom 4 H (m) 7,2 ppm 1 H (m) à 4,8 ppm).

**EXEMPLE 11: ACIDE $N_2$ (CHLORO-4 PHENYL) METHANE SULFONIQUE DE LA DIAMINO-2,4 (TRIMETHOXY-3,4,5 BENZYL)-5 PYRIMIDINE ET SON SEL DE SODIUM**

(III) : R = 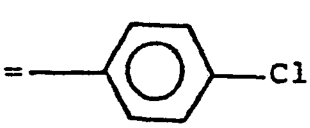

Y = H, Na

On ajoute successivement 14,5 g (0,05 mole) de triméthoprime et 7,0 g (0,05 mole) de chloro-4 benzaldéhyde dans 100 ml de pyridine. Aprés traitement selon la technique habituelle, on remet le produit brut en suspension dans environ 300 ml d'eau, et on améne à pH 8,5-9,0 par addition de soude diluée. On filtre un léger insoluble, puis on acidifie jusqu'à pH 2,20 par de l'acide chlorhydrique dilué. La forme acide du dérivé est filtrée, puis lavée soigneusement à l'eau puis à l'éthanol absolu. Aprés séchage, on obtient le dérivé recherché, forme acide à l'état pur. La CCM du produit présente un seul spot de Rf 0,62 dans le système A et de 0,56 dans le système B. La forme acide remise en suspension dans l'eau est convertie en sel de sodium par ajout de soude diluée jusqu'à pH 8,40-8,50. On évapore et on sèche pour obtenir la forme sodique du dérivé recherché, de pF 174°C (RMN:4 H (arom) (m) à 7,3 ppm, H (m) à 4,8 ppm).

**EXEMPLE 12: ACIDE $N_2$ (NITRO-4 PHENYL) METHANE SULFONIQUE DE LA DIAMINO-2,4 (TRIMETHOXY-3,4,5 BENZYL)-5 PYRIMIDINE ET SON SEL DE SODIUM**

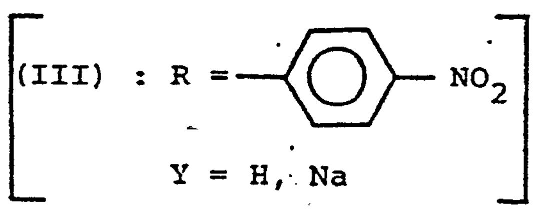

On introduit successivement 14,5 g de triméthoprime (0,05 mole) et 7,5 g d'aldéhyde nitro-4 benzoïque dans 100 ml de pyridine. Après traitement selon la technique habituelle, on remet en suspension le produit brut dans 400 ml d'eau et on alcalinise par de la soude diluée jusqu'à pH 10,0. On filtre un léger insoluble, puis on acidifie jusqu'à pH 4,0 par de l'acide chlorhydrique dilué. On filtre le précipite, puis on lave soigneusement à l'eau puis à l'éthanol absolu. Après séchage, on obtient à l'état pur le dérivé recherche sous sa forme acide. La CCM du produit ne donne qu'un spot de Rf 0,62 dans le système A et de 0,52 dans le système B. La forme acide est remise en suspension dans l'eau et dissoute par ajout de soude diluée jusqu'à pH 8,80. Apres évaporation de l'eau sous pression réduite et séchage, on obtient la forme sodique du derivé recherche de pF = 190°C. (RMN: 2 H (arom) (m) à 8,4 ppm, 2 H (arom) à 7,2 ppm, 1 H (m) à 4,8 ppm).

**EXEMPLE 13: ACIDE $N_2$ (NITRO-3 PHENYL) METHANE SULFONIQUE DE LA DIAMINO-2,4 (TRIMETHOXY-3,4,5 BENZYL)-5 PYRIMIDINE ET SON SEL DE SODIUM**

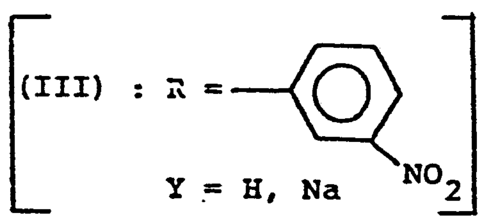

On introduit successivement 14,5 g (0,05 mole) de triméthoprime et 7,5 g (0,05 mole) d'aldéhyde nitro-3 benzoïque dans 100 ml de pyridine. Apres traitement selon la technique habituelle, on remet le produit brut en suspension dans l'eau, puis on alcalinise à la soude diluée jusqu'à pH 9,0. On filtre un léger insoluble, puis on acidifie jusqu'à pH 2,5 par de l'acide chlorhydrique dilué. On filtre le precipité, on lave à l'eau puis à l'éthanol. Apres séchage, on obtient la forme acide du dérivé recherché à l'état pur de pF 196°C. La CCM du produit donne un seul spot de Rf 0,56 dans le système A et de 0,46 dans le système B. La forme acide est remise en suspension à raison d'environ 3 % dans l'éthanol et redissoute par ajout de soude diluée 2 N jusqu'à pH 8,6-8,8. Apres évaporation et séchage, on obtient la forme sodique du dérivé recherche de pF 178°C. (RMN: 3H (arom) m à 8,4 ppm, 1 H (arom) à 7,2 ppm, 1 H (m) à 4,8 ppm).

**EXEMPLE 14: ACIDE $N_2$ (NITRO-2 PHENYL) METHANE SULFONIQUE DE LA DIAMINO-2,4 (TRIMETHOXY-3,4,5 BENZYL)-5 PYRIMIDINE ET SON SEL DE SODIUM**

(III) : R =
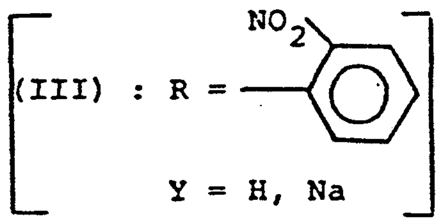
Y = H, Na

On introduit successivement 29 g (0,1 M) de triméthoprime, 15,1 g (0,1 M) d'aldéhyde orthonitrobenzoïque dans 100 ml de pyridine, puis on introduit en une heure trente 70 g d'anhydride sulfureux. Après traitement selon la technique habituelle, on remet le produit brut en suspension dans 400 ml d'eau et on amene à pH 8,80 par de la soude diluée. Après filtration d'un léger insoluble, on reprécipite par acidification avec de l'acide chlorhydrique dilué. On filtre le précipité, on lave à l'eau puis à l'éthanol. Après séchage, on obtient la forme acide du dèrivé recherché à l'état pur de pF 180° C. La CCM du produit donne un seul spot de Rf 0,55 dans le système A et de 0,45 dans le système B. La forme acide est remise en suspension dans l'éthanol et redissoute par ajout de soude jusqu'à pH 8,80. Après évaporation et séchage, on obtient la forme sodique du dérivé recherché de pF = 173° C (RMN: arom (m) 4 H à 7,8 ppm. -H (m) à 4,8 ppm).

**EXEMPLE 15: ACIDE $N_2$ (METHOXY-2 PHENYL) METHANE SULFONIQUE DE LA DIAMINO-2,4 (TRIMETHOXY-3,4,5 BENZYL)-5 PYRIMIDINE ET SON SEL DE SODIUM**

(III) : R =
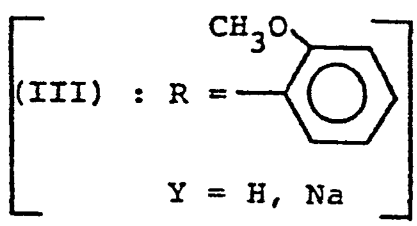
Y = H, Na

On ajoute successivement 14,5 g (0,05 mole) de triméthoprime et 6,8 g (0,05 mole) d'aldéhyde ortho anisique à 100 ml de pyridine. Après le traitement habituel, le produit brut est remis en suspension dans l'eau et redissous par ajout de soude diluée jusqu'à pH 7,70-7,90. On filtre un léger insoluble, puis on acidifie vers pH 2,2 par de l'acide chlorhydrique dilué. Le précipité est filtré, lavé à l'eau, puis abondamment à l'éthanol absolu tiède; après séchage, on obtient la forme acide pure du dérivé recherché de pF 150° C. La CCM du produit donne un seul spot de Rf 0,58 dans le système A et de 0,44 dans le système B. Par traitement à la soude diluée jusqu'à pH 8,8-8,9 de la forme acide, on obtient le sel de sodium qui est isolé par èvaporation de la solution (pF = 165°C). (RMN: $OCH_3$ (s) 3 H à 3,9 ppm, arom (m) 4 H à 7,2 ppm - H (m) à 4,8 ppm).

**EXEMPLE 16: ACIDE $N_2$ (METHOXY-3 PHENYL) METHANE SULFONIQUE DE LA DIAMINO-2,4 (TRIMETHOXY-3,4,5 BENZYL)-5 PYRIMIDINE ET SON SEL DE SODIUM**

(III) : R =
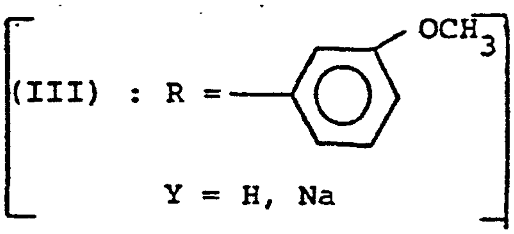
Y = H, Na

On ajoute successivement 14,5 g (0,05 mole) et 6,8 g (0,05 mole) d'aldéhyde méta anisique à 100 ml de pyridine, puis on traite de façon habituelle. Le produit brut est remis en suspension dans 400 ml d'eau et dissous par ajout de soude diluée jusqu'à pH 8,10-8,20. Après filtration d'un léger insoluble, on revient en milieu acide par de l'acide chlorhydrique dilué jusqu'à pH 2,4. La forme acide précipite, on la filtre et on lave à l'eau puis à l'éthanol absolu. Après séchage, on obtient la forme acide pure du dérivé recherché. La CCM du produit donne un seul spot de Rf 0,61 dans le système A et de 0,56 dans le système B. La forme acide est remise en suspension dans l'eau et traitée par de la soude diluée jusqu'à pH 8,10-8,20. La solution est évaporée à sec sous pression réduite; aprés séchage, on obtient la forme sodique du dérivé recherché de pF 192°C. (RMN: 4 H (arom) (m) 7,2 ppm.-$OCH_3$ (3 H) (s) 4,0 ppm, H (m) à 4,8 ppm).

**EXEMPLE 17: ACIDE $N_2$ (TRIMETHOXY-3,4,5 PHENYL)METHANE SULFONIQUE DE LA DIAMINO-2,4 (TRIMETHOXY-3,4,5 BENZYL)-5 PYRIMIDINE ET SON SEL DE SODIUM**

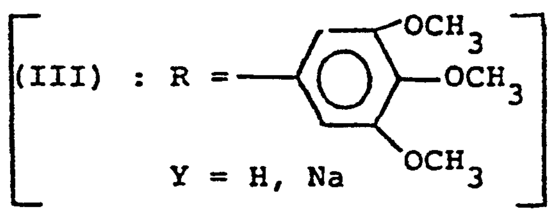

On ajoute successivement 14,5 g (0,05 mole) de triméthoprime et 9,3 g (0,05 mole) de triméthoxy-3,4,5 benzaldéhyde dans 100 ml de pyridine. Après le traitement habituel, le produit brut est remis en suspension aqueuse et traité par de la soude diluée jusqu'à pH 9,0. On filtre éventuellement un léger insoluble, puis on acidifie le filtrat jusqu'à pH 2,5 par addition d'acide chlorhydrique dilué. Le précipité est filtré, lavé soigneusement à l'eau, puis à l'éthanol chaud. Après séchage, on obtient la forme acide pure du dérivé recherché. La CCM du produit donne un seul spot de Rf 0,65 dans le système A et de 0,49 dans le système B. La forme acide peut être traitée dans l'eau par de la soude diluée jusqu'à pH 9,0. Par évaporation de la solution sous pression réduite, puis séchage, on obtient la forme sodique du dérivé recherché de pF 188°C. (RMN 2 H (arom) 6,60 (s) - 6 H (s)-$OCH_3$ à 3,70 ppm 3 H (s)-$OCH_3$ à 3,60 ppm, H (m) à 4,8 ppm).

**EXEMPLE 18: ACIDE $N_2$ (METHYLENE DIOXY-3,4 PHENYL)METHANE SULFONIQUE DE LA DIAMINO-2,4 (TRIMETHOXY-3,4,5 BENZYL)-5 PYRIMIDINE ET SON SEL DE SODIUM**

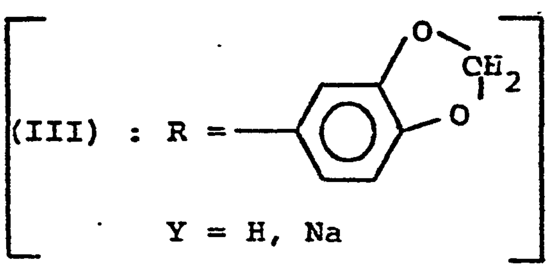

On introduit successivement 14,5 g (0,05 mole) de triméthoprime et 7,5 g (0,05 mole) de pipéronal dans 100 ml de pyridine. Après traitement selon la technique habituelle, on remet le produit en suspension dans 300 ml d'eau et on alcalinise par de la soude diluée jusqu'à pH 9,0. On filtre un léger insoluble, puis on acidifie par de l'acide chlorhydrique dilué jusqu'à pH 2,5. Le précipité apparu est filtre, puis lavé abondamment à l'eau puis à l'éthanol. Après séchage, on obtient la forme acide du dérivé recherché. La CCM du produit donne un seul spot de Rf 0,53 dans le système A et de 0,44 dans le système B. On peut traiter la suspension aqueuse de la forme acide par ajout de soude diluée jusqu'à pH 8,90-9,0. La solution limpide est évaporée sous pression réduite, puis séchée pour donner la forme sodique du dérivé recherché, de pF 165°C. (RMN: 3 H (arom) m à 7,2 ppm -$CH_2$-2H (s) à 6,0 ppm - 1 H (m) à 4,8 ppm).

**EXEMPLE 19: ACIDE $N_2$ FURYL-2 METHANE SULFONIQUE DE LA DIAMINO-2,4 (TRIMETHOXY-3,4,5 BENZYL)-5 PYRIMIDINE ET SON SEL DE SODIUM**

(III) : R = 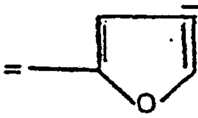

Y = H, Na

On ajoute successivement 39,0 g (0,4 mole) de furaldéhyde et 116 g (0,4 mole) de triméthoprime à 800 ml de pyridine, puis on fait barbotter de l'anhydride sulfureux à raison de 80 g/h pendant 4 heures. La température monte spontanément de 20 à 55°C. Après refroidissement, on coule sur 5 litres d'éther et on filtre le précipité. Le produit brut séché est remis en suspension dans 12 volumes d'eau et traité par de la soude diluée jusqu'à pH 8,70. Après filtration d'un léger insoluble, on acidifie le filtrat par de l'acide chlorhydrique dilué jusqu'à pH 2,5. On filtre la forme acide insoluble et on la lave soigneusement à l'eau puis à l'éthanol. Après séchage, on obtient la forme acide du dérivé recherché de pF 163°C. La CCM du produit donne un seul spot de Rf 0,46 dans le système A et de 0,32 dans le système B. Par traitement à la soude diluée de la suspension aqueuse de cette forme acide jusqu'à pH 8,70, on obtient après évaporation sous pression réduite et séchage, la forme sodique du dérivé recherché de pF; 125°C (RMN:3 H (arom) (m) 6,5 et 7,5 ppm - CH - 1H (m) à 4,8 ppm).

**EXEMPLE 20: ACIDE $N_2$ (THIENYL-2) METHANE SULFONIQUE DE LA DIAMINO-2,4 (TRIMETHOXY-3,4,5 BENZYL)-5 PYRIMIDINE ET SON SEL DE SODIUM**

(III) : R = 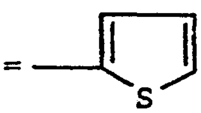

Y = H, Na

On ajoute successivement 14,5 g (0,05 mole) de triméthoprime et 5,6 g (0,05 mole) d'aldéhyde 2 thénoïque à 100 ml de pyridine, puis on traite conformément à l'Exemple 6.

Le produit brut est remis en suspension aqueuse et traité par de la soude diluée jusqu'à pH 8,40-8,60. Après filtration d'un léger insoluble, on traite le filtrat par de l'acide chlorhydrique dilué jusqu'à pH 2,5. Le précipité apparu est filtré, lavé à l'eau, puis à l'éthanol. Après séchage, on obtient la forme acide du dérivé recherché à l'état pur. La CCM du produit donne un seul spot de Rf 0,61 dans le système A et de 0,43 dans le système B. Par traitement de la suspension aqueuse de la forme acide par de la soude diluée jusqu'à pH 8,4-8,60, on obtient après évaporation et séchage, le sel sodique du dérivé recherche de pF 152°C (RMN: arom 2 H (m) à 7,2 ppm - 1 H (m) à 7,8 ppm; H (m) à 4,8 ppm).

**EXEMPLE 21: ACIDE $N_2$ (HYDROXY-4 METHOXY-3) PHENYL METHANE SULFONIQUE DE LA DIAMINO-2,4 (TRIMETHOXY-3,4,5 BENZYL)-5 PYRIMIDINE ET SON SEL DE SODIUM**

(III) : R = 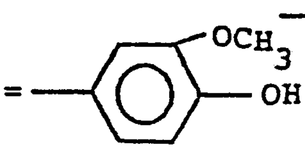

Y = H, Na

On ajoute successivement 14,5 g (0,05 mole) de triméthoprime et 7,5 g (0,05 mole) de vanilline à 100 ml de pyridine, puis on traite conformément à l'Exemple précédent. Le produit brut est remis en suspension aqueuse et traité par de la soude diluée jusqu'à pH 7,80-8,00. On filtre un léger insoluble, puis on reprécipite la forme acide par addition d'acide chlorhydrique dilué jusqu'à pH 2,5. Le précipité est filtré, lavé soigneusement à l'eau puis à l'éthanol. Après séchage, on obtient la forme acide pure. La CCM du produit donne un seul spot de Rf 0,39 dans le système A et de 0,27 dans le système B. Par traitement de la suspension aqueuse de la forme acide par de la soude diluée jusqu'à pH 7,70, on obtient, après évaporation et séchage, la forme sodique du dérivé recherché de pF 162°C (RMN: $OCH_3$ (s) 3 H à 3,9 ppm, arom (m) 4 H à 7 ppm, H (m) à 4,8 ppm, OH (s) à 8,5 ppm).

**EXEMPLE 22: ACIDE $N_2$ PYRIDYL-2 METHANE SULFONIQUE DE LA (TRIMETHOXY-3,4,5 BENZYL)-5 DIAMINO-2,4 PYRIMIDINE**

(III) : R =

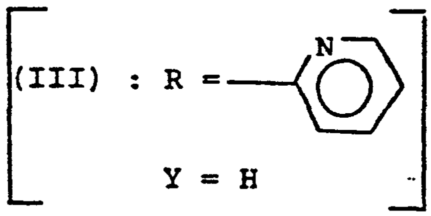

Y = H

On introduit successivement 14,5 g (0,05 mole) de triméthoprime et 5,5 g (0,05 mole) de pyridine 2 carboxaldéhyde dans 100 ml de pyridine, puis on traite suivant l'Exemple 6. Le produit brut est ensuite dispersé dans l'eau et acidifié avec précaution jusqu'à pH 2,8 avec de l'acide chlorhydrique dilué. On filtre un léger insoluble et le filtrat est ramené lentement à pH 5,60 par de la soude diluée. La forme acide sulfonique du dérivé recherché précipite à l'état pur; après filtration, lavage à l'eau et à l'éthanol, on obtient le dérivé recherché à l'état pur de pF 185°C. La CCM du produit donne un seul spot de Rf 0,45 dans le système A et de 0,2 dans le système B. Le dérivé recherché insoluble à pH 5,6 a un caractère amphotère et peut être mis en solution à pH légèrement acide entre pH 3 et pH 5 en pH légèrement alcalin entre pH 7 et pH 9. (RMN:5 H (arom) (m) à 6,8-7,2 ppm -CH-1H à 4,8 ppm).

**EXEMPLE 23: ACIDE $N_2$ CYCLOHEXYL METHANE SULFONIQUE DE LA (TRIMETHOXY-3,4,5 BENZYL) -5 DIAMINO-2,4 PYRIMIDINE ET SON SEL DE SODIUM**

(III) : R =

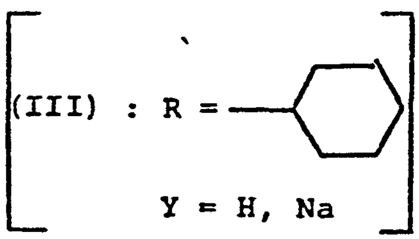

Y = H, Na

On ajoute successivement 14,5 g (0,05 mole) de triméthoprime et 5,6 g (0,05 mole) de cyclohexane-carboxaldéhyde à 100 ml de pyridine, puis on traite selon l'Exemple 6. La suspension aqueuse du produit brut est traitée ensuite par de la soude diluée jusqu'à pH 10. Après filtration d'un léger insoluble, on acidifie rapidement le filtrat jusqu'à pH 3,0. Après filtration du précipité et lavages à l'eau et à l'éthanol chaud, on obtient après séchage, la forme acide du dérivé recherché de pF 162°C. La CCM du produit donne un seul spot de Rf 0,9 dans le système A et de 0,55 dans le système B.

On peut obtenir la forme sodique du dérivé recherché par traitement de la suspension aqueuse de la forme acide par de la soude diluée jusqu:à pH 9,0, suivi par l'évaporation à basse température de la solution, et séchage (RMN:11 (H) (m) à 1,5-2,0 ppm, CH (1H) (m) à 4,8 ppm).

**EXEMPLE 24: ACIDE $N_2$ (ETHOXY-3 HYDROXY-4) PHENYL METHANE SULFONIQUE DE LA (TRIMETHOXY-3,4,5 BENZYL)-5 DIAMINO-2,4 PYRIMIDINE ET SES SELS DE METAUX ALCALINS OU D'AMINES ORGANIQUES**

(III) : R =

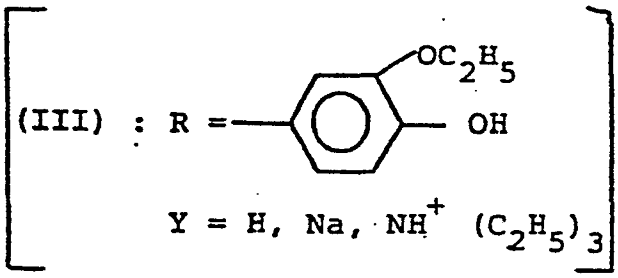

Y = H, Na, $NH^+ (C_2H_5)_3$

$NH^+ (C_2H_4OH)_3$,$NH_2^+ (C_2H_4OH)_2$

$NH_3^+ (C_2H_4OH)$

a)-(Y = H)

On ajoute successivement 145 g (0,5 mole) de triméthoprime et 83 g (0,5 mole) d'éthyl vanilline à 500 ml de pyridine, et on introduit environ 130 g d'anhydride sulfureux en trois heures. La température de mélange réactionnel monte spontanément entre 40 et 50°C dès le début de l'introduction et s'y maintient jusqu'à la fin de la réaction. On abandonne 24 heures à température ordinaire. On verse ensuite le milieu réactionnel dans un grand volume d'éther, le précipité apparu est filtré et lavé à l'éther. Le produit brut est alors dispersé dans l'eau et traité par de la soude diluée jusqu'à pH 9,30-9,40. On filtre un léger insoluble, puis on reprécipite le produit par addition d'acide chlorhydrique à la solution alcaline jusqu'à pH 2-3. On filtre, on lave à l'eau puis à l'éthanol. Après séchage, on obtient la forme acide du dérivé recherché de pF 145°C. La CCM du produit donne un seul spot de Rf 0,50 dans le système A et de Rf 0,40 dans le système B.

b)-(Y = Na)

On peut obtenir le sel de sodium du produit par traitement de la suspension aqueuse de la forme acide par de la soude diluée jusqu'à pH 8,80. Après évaporation de la solution sous pression réduite et séchage, on obtient le sel de sodium du dérivé recherché de pF 170°C

(RMN: 1 H (s) à 8,5 ppm - 3 H (arom) (m) à 7,2 ppm - 2 H -$OCH_2$ (q) à 3,9 ppm - 3 H (t) $CH_3$ à 1 ppm).

c)-(Y = $NH^+ (C_2H_5)_3$)

Le sel de triéthylamine du dérivé recherché peut être obtenu par traitement de la suspension hydroalcoolique (50/50) de la forme acide par la triéthylamine pure jusqu'à pH 8,90-9,0. La solution limpide est évaporée sous pression réduite. Après séchage,on obtient le sel de triéthylamine du dérivé recherché de pF 120°C.

d)-(Y = $NH_3^+ (C_2H_4OH)$)

On peut obtenir le sel de monoéthanolamine en traitant 10 g de forme acide en suspension dans 100 ml d'éthanol aqueux à 50 % par un léger excès de monoéthanolamine. La solution résultante est évaporée à sec sous pression réduite. Le résidu est repris dans un mélange acétone benzène. pF 120°C (dec).

e)-(Y = $NH_2^+ (C_2H_4OH)_2$)

On peut obtenir le sel de diéthanolamine en traitant comme ci-dessus la forme acide par un léger excès de diéthanolamine; on obtient le sel de pF 125°C (dec).

f)-(Y = $NH^+ (C_2H_4OH)_3$)

On obtient de même, le sel de triéthanolamine en traitant la forme acide selon d) par un léger excés de triéthanolamine; on obtient ainsi le sel de pF 107°C.

**EXEMPLE 25: ACIDE $N_2$ (HYDROXY-2) PHENYL METHANE SULFONIQUE DE LA (TRIMETHOXY-3,4,5 BENZYL)-5 DIAMINO-2,4 PYRIMIDINE ET SELS DE METAUX ALCALINS OU SES SELS D'AMINES**

(III) : R =

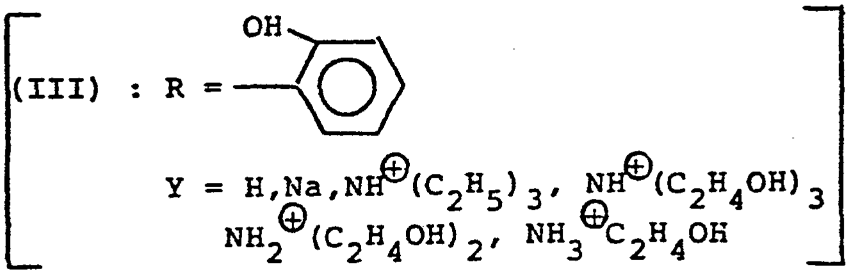

Y = H,Na,$NH^{\oplus}(C_2H_5)_3$, $NH^{\oplus}(C_2H_4OH)_3$

$NH_2^{\oplus}(C_2H_4OH)_2$, $NH_3^{\oplus}C_2H_4OH$

a) (Y = H)

On ajoute successivement 29,0 g (0,1 mole) de triméthoprime et 13,0 g de salicylaldéhyde à 170 ml de pyridine, puis on introduit 80 à 90 g d'anhydride sulfureux en trois heures environ. La température du milieu réactionnel se maintient spontanément entre 40 et 50°C. Après 24 heures à température ordinaire, le milieu est coulé dans l'éther en excès, le précipité résultant est filtré, lavé à l'éther et séché. On remet le produit brut en suspension aqueuse et on alcalinise jusqu'à pH 9,30 par de la soude diluée. Après filtration de l'insoluble, on acidifie le filtrat jusqu'à pH 2,0 par de l'acide chlorhydrique dilué, la forme acide précipite. On filtre, on lave abondamment à l'eau, puis à l'éthanol. Après séchage, on obtient la forme acide du dérivé recherché de pF 145°C. La CCM du produit donne un seul spot de Rf 0,40 dans le système A et de Rf 0,30 dans le système B.

b) - (Y = Na)

On peut obtenir le sel de sodium du dérivé recherché par traitement de la suspension aqueuse de la forme acide par de la soude diluée jusqu'à pH 8,90. Après évaporation et séchage, on obtient le sel de sodium du dérivé recherché.

c) - (Y = $NH^+(C_2H_5)_3$)

On peut obtenir le sel de triéthylamine du produit en traitant la suspension hydroalcoolique (50/50) de la forme acide par de la triéthylamine jusqu'à pH 8,90. Après évaporation de la solution sous pression réduite, puis séchage, on obtient le sel de triéthylamine du dérivé recherché (pF 113°C).

d) - (Y = $NH_3^+(C_2H_4OH)$)

On obtient le sel de monoéthanolamine en traitant 10 g de la forme acide dans 100 ml d'éthanol aqueux à 50 % avec un léger excès de monoéthanolamine. Après évaporation sous pression réduite et reprise à l'acétone, on obtient le sel de monoéthanolamine du dérivé recherché pF 80/90°C (dec).

e) - (Y = $NH_2^+(C_2H_4OH)_2$)

Le sel de diéthanolamine est obtenu selon le procédé ci-dessus en utilisant un léger excès de diéthanolamine pF 100/110°C (dec).

f) - (Y = $NH^+ (C_2H_4OH)_3$)

Le sel de triéthanolamine est obtenu selon d), en utilisant la triéthanolamine en léger excès pF 110°C (dec).

Les analyses élémentaires des éléments suivants ont été effectuées pour tous les produits cités: C, H, N, S et Na. Ces analyses correspondent toutes (à 0,3 % près) aux données théoriques.

Parmi les signaux de spectrographie par résonance magnétique nucléaire du $^1H$, on n'a cité que les signaux caractéristiques du Radical R. Les signaux du motif commun étant les suivants (solution/DMSO $d^6$ des sels de sodium par rapport au T.M.S.) 7,53 ppm 1 H (s) aromatique de la pyrimidine; 6,55 ppm 2 H (s) aromatiques; 6,44 ppm 2 H (s large) du $NH_2$; 5,90 ppm 1 H (s large) du NH; 3,75 ppm 6 H (s)$-$ $OCH_3$ en 3 et 5;3,64 ppm 3 H (s)$-OCH_3$ en 4; 3,54 ppm 2 H (s)$-CH_2-$.

## COMPTE-RENDU DES ESSAIS PHARMACOLOGIQUES EFFECTUES AVEC LES PRODUITS CONFORMES A L'INVENTION

Les études pharmacologiques ont porté d'une part sur la toxicité, et d'autre part sur l'activite antibactérienne.

**A - Toxicité**

Du point de vue de la toxicité immédiate, il ressort que les dérivés selon l'invention sont très peu toxiques et surtout beaucoup moins que les diamino-2,4 pyrimidines initiales. Les doses létales (DL 50) obtenues par administration orale ou intraveineuse chez la souris sont très élevées.

Les valeurs comparatives rassemblées dans le Tableau I ci-après exemplifient ces différences de toxicité; les mesures ont été réalisées sur des lots homogènes de 10 souris femelles SWISS IOPS, âgées de 4 semaines et d'un poids moyen de 18 à 20 g.

**TABLEAU I**

| DL 50 mg/kg | Triméthoprime | Dérivé de l'ex. 18 | Dérivé de l'ex. 15 f | Dérivé de l'ex. 24 | Dérivé de l'ex. 19 |
|---|---|---|---|---|---|
| Per os | 4 000 | > 12 000 | > 12 000 | > 12 000 | > 12 000 |
| I.V. | 120 | | 1 000 | 1 000 | 900 |

**B - Activité antibactérienne**

L'activité antibactérienne des dérivés selon l'invention a été testée in vitro en déterminant les concentrations minimales inhibitrices (C.M.I.) sur différents germes. Les valeurs comparées des C.M.I. des divers composés rapportées à celles de la triméthoprime pris comme référence, ainsi que le rapport R représentant

$$\text{activité} \times \frac{\text{Masse molaire dérivé}}{\text{Masse molaire triméthoprime}}$$

ont été rassemblées dans le Tableau II ci-après.

**TABLEAU II**

| EXEMPLE | MILIEU OXOIDE COLI 4 | | MILIEU MULLER HINTON COLI 4 | | MILIEU MULLER HINTON BACIL PUMILUS | |
|---|---|---|---|---|---|---|
| | % activité par rapport à la base | % activité par rapport à la base corrigée de la masse molaire | % activité par rapport à la base | % activité par rapport à la base corrigée de la masse molaire | % activité par rapport à la base | % activité par rapport à la base corrigée de la masse molaire |
| Exemple 1 | 17,1 | 24 | 0,3 | 0,4 | 0,1 | — |
| Exemple 2 | 4 | 5,8 | 4,3 | 6,2 | 2,8 | 4 |
| Exemple 3 | 18,35 | 28 | 4,55 | 10,8 | 2,9 | 6,8 |
| Exemple 4 | 12,9 | 20 | 14,1 | 22 | 11,8 | 18 |
| Exemple 5 | 18,4 | 29 | 23,3 | 37 | 19,4 | 30 |
| Exemple 6 | 18,8 | 32 | 15,75 | 27 | 16,6 | 28 |
| Exemple 7 | 14 | 23 | 15,5 | 25 | 14 | 23 |
| Exemple 8 | 13,3 | 21 | 10,2 | 16,4 | 18,4 | 26 |
| Exemple 9 | 14,7 | 26 | 13,3 | 23 | 14,1 | 25 |
| Exemple 10 | 14,1 | 25 | 11,6 | 20 | 10,4 | 18 |
| Exemple 11 | 25,6 | 45 | 17,6 | 31 | 23 | 41 |
| Exemple 12 | 11,7 | 21 | 18,1 | 32 | 11,9 | 22 |
| Exemple 13 | 32,5 | 59 | 17,2 | 31 | 23,2 | 42 |
| Exemple 14 | 10,6 | 19 | 13,9 | 25 | 11,0 | 20 |
| Exemple 15 | 64 | 107 | 47 | 73 | 69 | 106 |
| Exemple 16 | 13,6 | 23 | 16,8 | 29 | 10,7 | 19 |
| Exemple 17 | 34,8 | 68 | 24,3 | 48 | 42,7 | 84 |
| Exemple 18 | 27 | 48 | 14,85 | 26 | 21,4 | 38 |
| Exemple 19 | 58,4 | 95 | 69,4 | 112 | 49,5 | 80 |
| Exemple 20 | 62,6 | 105 | 73,5 | 123 | 83,4 | 140 |
| Exemple 21 | 49,8 | 90 | 43,3 | 79 | 34,6 | 63 |
| Exemple 24b | 48,7 | 93 | 43,9 | 84 | 42,2 | 79 |
| Exemple 24d | 13,1 | 26 | 23,2 | 46 | 37,0 | 74 |
| Exemple 24e | 21,3 | 46 | 40,0 | 86 | 40,0 | 86 |
| Exemple 24f | 27,4 | 63 | 40,3 | 92 | 31,8 | 73 |
| Exemple 25c | | | 57 | 116 | | |
| Exemple 25d | 28,3 | 53 | 45,4 | 85 | 55,5 | 104 |
| Exemple 25e | 36,6 | 74 | 45,8 | 93 | 75,8 | 154 |
| Exemple 25f | 38,3 | 84 | 55,4 | 120 | 73,1 | 159 |

Les composés selon l'invention sont des antibactériens puissants; de plus, ils potentialisent l'action antibactérienne propre des sulfamides dans le traitement des affections bacteriennes en particulier dans les domaines respiratoire, digestif, urinaire ou otorhynolaryngologique. A titre d'exemple, on peut citer les associations avec des sulfamides comme la sulfadiazine, la sulfamonométhoxine, la sulfadiméthoxine, le sulfaméthoxazol, le sulfamoxol, le sulfa-2 diméthyl-2,4 isoxazole et la sulfanilamido-4 diméthoxy-5,6 pyrimidine. Les composés de formule III peuvent être combinés avec les sulfamides précités en proportions variables allant de 1:1 à 1:5. La dose unitaire de principe actif correspondant aux produits de formule III peut aller de 50 à 1000 mg.

Ainsi que cela ressort de ce qui précède, l'invention ne se limite nullement à ceux de ses modes de mise en oeuvre, de réalisation et d'application qui viennent d'être décrits de façon plus explicite; elle en embrasse, au contraire, toutes les variantes qui peuvent venir à l'esprit du technicien en la matière, sans s'écarter du cadre, ni de la portée, de la présente invention.

**Revendications**

1°. Dérivés solubles $N_2$ substitués de la diamino-2,4 benzyl-5 pyrimidine répondant à la formule III ci-aprés:

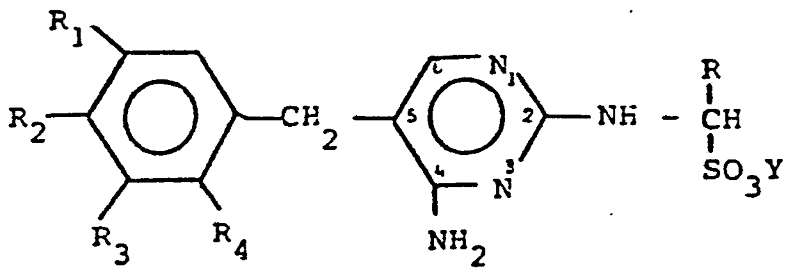

(III)

dans laquelle:

$R_1$, $R_2$, $R_3$, $R_4$ qui peuvent être identiques ou différents, représentent un atome d'hydrogène, un atome d'halogène ou un groupe alkyle, thioalkyle, alcoxy-,benzyloxy- ou alkyloxyalcoxy-,

Y représente un atome d'hydrogène, un métal alcalin ou une base organique pharmaceutiquement compatible, et

R représente un atome d'hydrogène ou un radical alkyle linéaire ou ramifié comprenant de 1 à 7 atomes de carbone,un radical cycloalkyle comprenant de 5 à 8 atomes de carbone, un radical phényl éventuellement substitué par des substituants tels que, halogène, nitro-, hydroxyle, dialkylamino-, alcoxy-, alkyl de $C_1$ à $C_3$,deux substituants adjacents pouvant constituer un cycle alkyldioxy-, le furanne, le thiophène, la pyridine.

2°- Dérivé selon la Revendication 1, caractérisé en ce que le substituant sur le $N_2$,est le radical méthane sulfonique ou son sel de sodium.

3°- Dérivé selon la Revendication 1, caractérisé en ce que le substituant sur le $N_2$,est le radical éthane sulfonique ou son sel de sodium.

4°- Dérivé selon la Revendication 1, caractérisé en ce que le substituant sur le $N_2$, est le radical n-butane sulfonique.

5°- Dérivé selon la Revendication 1, caractérisé en ce que le substituant sur le $N_2$, est le radical méthyl-2 propane sulfonique.

6°- Dérivé selon la Revendication 1, caractérisé en ce que le substituant sur le $N_2$, est le radical diméthyl-2,2 propane sulfonique.

7°- Dérivé selon la Revendication 1, caractérisé en ce que le substituant sur le $N_2$, est le radical diméthyl-2,3 pentane sulfonique.

8°- Dérivé selon la Revendication 1, caractérisé en ce que le substituant sur le $N_2$, est le radical éthyl-2 butane sulfonique.

9°- Dérivé selon la Revendication 1, caractérisé en ce que le substituant sur le $N_2$, est le radical phényl méthane sulfonique.

10°- Dérivé selon la Revendication 1, caractérisé en ce que le substituant sur le $N_2$, est le radical (chloro-2) phényl méthane sulfonique.

11°- Dérivé selon la Revendication 1, caractérisé en ce que le substituant sur le $N_2$, est le radical (chloro-3) phényl méthane sulfonique.

12°- Dérivé selon la Revendication 1, caractérisé en ce que le substituant sur le $N_2$, est le radical (chloro-4) phényl méthane sulfonique.

13°- Dérivé selon la Revendication 1, caractérisé en ce que le substituant sur le $N_2$, est le radical (nitro-4) phényl méthane sulfonique.

14°- Dérivé selon la Revendication 1, caractérisé en ce que le substituant sur le $N_2$, est le radical (nitro-3) phényl méthane sulfonique.

15°- Dérivé selon la Revendication 1, caractérisé en ce que le substituant sur le $N_2$, est le radical (nitro-2) phényl méthane sulfonique.

16°- Dérivé selon la Revendication 1, caractérisé en ce que le substituant sur le $N_2$, est le radical (méthoxy-2) phényl méthane sulfonique.

17°- Dérivé selon la Revendication 1, caractérisé en ce que le substituant sur le $N_2$, est le radical (méthoxy-3) phényl méthane sulfonique.

18°- Dérivé selon la Revendication 1, caractérisé en ce que le substituant sur le $N_2$, est le radical (tri-méthoxy-3,4,5) phényl méthane sulfonique.

19°- Dérivé selon la Revendication 1, caractérisé en ce que le substituant sur le $N_2$, est le radical (méthyl-ènedioxy-3,4) phényl méthane sulfonique.

20°- Dérivé selon la Revendication 1, caractérisé en ce que le substituant sur le $N_2$, est le radical furyl-2 méthane sulfonique ou son sel de sodium.

21°- Dérivé selon la Revendication 1, caractérisé en ce que le substituant sur le $N_2$, est le radical thiényl-2 méthane sulfonique ou son sel de sodium.

22°- Dérivé selon la Revendication 1, caractérisé en ce que le substituant sur le $N_2$, est le radical (méthoxy-2 hydroxy-4) phényl méthane sulfonique.

23° - Dérivé selon la Revendication 1, caractérisé en ce que le substituant sur le $N_2$, est le radical pyridyl-2 méthane sulfonique ou ses sels acides ou basiques.

24°- Dérivé selon la Revendication 1, caractérisé en ce que le substituant sur le $N_2$, est le radical cyclohexylméthane sulfonique ou son sel de sodium.

25°- Dérivé selon la Revendication 1, caractérisé en ce que le substituant sur le $N_2$, est le radical (éthoxy-3 hydroxy-4) phényl méthane sulfonique, son sel de sodium ou ses sels d'amines.

26°- Dérivé selon la Revendication 1, caractérisé en ce que le substituant sur le $N_2$, est le radical (hydroxy-2) phényl méthane sulfonique, son sel de sodium ou ses sels d'amines.

27°- Procédé de préparation des produits selon la Revendication 1, caractérisé en ce que l'on fait réagir dans un solvant approprié tel que la pyridine, notamment, un dérivé de diamino-2,4 benzyl-5 pyrimidine, avec un aldéhyde et l'anhydride sulfureux, puis en ce qu'on isole le produit de la réaction.

28°- Procédé selon la Revendication 2, caractérisé en ce que les quantités de diamino-2,4 benzyl-5 pyrimidine et d'aldéhyde, sont stoechiométriques, tandis que l'anhydride sulfureux est utilisé en excés.

29°- Procédé selon l'une quelconque des Revendications 2 et 3, caractérisé en ce que le produit de la réaction est isolé par dilution du milieu réactionnel par un solvant miscible à la pyridine, tel que l'éther ou un hydrocarbure.

30°- Procédé selon l'une quelconque des Revendications 2 à 4, caractérisé en ce que le produit isolé est en outre purifié par lavage à l'alcool aqueux.

31°- Médicament utilisable en médecine humaine et/ou vétérinaire, caractérisé en ce qu'il contient au moins un composé selon les Revendications 1 à 26 à l'état pur ou en association avec un ou plusieurs autres principes actifs et un ou plusieurs adjuvants ou diluants compatibles et pharmaceutiquement acceptables.

**Patentansprüche**

1. Neue lösliche Derivate vom Diamino-2,4-benzyl-5-pyrimidin mit $N_2$-Substitution gemäß Formel III:

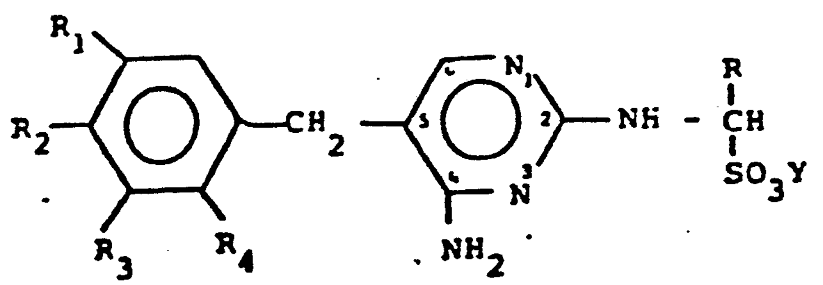

(III)

worin

$R_1, R_2, R_3, R_4$ gleich oder verschieden sein können und für ein Wasserstoffatom oder ein Halogenatom stehen oder für eine Alkylgruppe, eine Thioalkylgruppe, eine Alkoxygruppe, eine Benzyloxygruppe oder eine Alkyloxyalkoxygruppe;

Y für ein Wasserstoffatom steht oder ein Alkalimetallatom oder eine pharmazeutisch akzeptable organische Base und

R für ein Wasserstoffatom oder einen linearen oder verzweigten Alkylrest mit 1 bis 7 Kohlenstoffatomen oder einen Cycloalkylrest mit 5 bis 8 Kohlenstoffatomen, oder einen Phenylrest, welcher gegebenenfalls substituiert ist mit Substituenten wie Halogen, Nitro, Hydroxyl, Dialkylamino, Alkoxy, Alkyl mit 1 bis 3 Kohlenstoffatomen, wobei zwei benachbarte Substituenten eine Cycloalkyldioxygruppe bilden können, Furan, Thiophen oder Pyridin.

2. Derivat nach Anspruch 1, dadurch gekennzeichnet, daß es sich bei dem Substituenten an der $N_2$-Stellung um einen Methansulfonsäurerest handelt oder um dessen Natriumsalz.

3. Derivat nach Anspruch 1, dadurch gekennzeichnet, daß es sich bei dem Substituenten in $N_2$-Stellung um einen Äthansulfonsäurerest oder um dessen Natriumsalz handelt.

4. Derivat nach Anspruch 1, dadurch gekennzeichnet, daß es sich bei dem Substituenten in $N_2$-Stellung um einen N-Butansulfonsäurerest handelt.

5. Derivat nach Anspruch 1, dadurch gekennzeichnet, daß der Substituent in $N_2$-Stellung ein Methyl-2-Propansulfonsäurerest ist.

6. Derivat nach Anspruch 1, dadurch gekennzeichnet, daß der Substituent in $N_2$-Stellung der Dimethyl-2,2,-Propansulfonsäurerest ist.

7. Derivat nach Anspruch 1, dadurch gekennzeichnet, daß der Substituent in $N_2$-Stellung der Dimethyl-2,3-Pentansulfonsäurerest ist.

8. Derivat nach Anspruch 1, dadurch gekennzeichnet, daß der Substituent in $N_2$-Stellung der Äthyl-2-Butansulfonsäurerest ist.

9. Derivat nach Anspruch 1, dadurch gekennzeichnet, daß der Substituent in $N_2$-Stellung der Phenylmethansulfonsäurerest ist.

10. Derivat nach Anspruch 1, dadurch gekennzeichnet, daß der Substituent in $N_2$-Stellung der (Chlor-2)-Phenylmethansulfonsäurerest ist.

11. Derivat nach Anspruch 1, dadurch gekennzeichnet, daß der Substituent in $N_2$-Stellung der (Chlor-3)-Phenyl-methansulfonsäurerest ist.

12. Derivat nach Anspruch 1, dadurch gekennzeichnet, daß der Substituent in $N_2$-Stellung der (Chlor-4)-Phenylmethansulfonsäurerest ist.

13. Derivat nach Anspruch 1, dadurch gekennzeichnet, daß der Substituent in $N_2$-Stellung der (Nitro-4)-Phenyl-methansulfonsäurerest ist.

14. Derivat nach Anspruch 1, dadurch gekennzeichnet, daß der Substituent in $N_2$-Stellung der (Nitro-3)-Phenyl-methansulfonsäurerest ist.

15. Derivat nach Anspruch 1, dadurch gekennzeichnet, daß der Substituent in $N_2$-Stellung der (Nitro-2)-Phenyl-methansulfonsäurerest ist.

16. Derivat nach Anspruch 1, dadurch gekennzeichnet, daß der Substituent in $N_2$-Stellung der (Methoxy-2)-Phenylmethansulfonsäurerest ist.

17. Derivat nach Anspruch 1, dadurch gekennzeichnet, daß der Substituent in $N_2$-Stellung der (Methoxy-3)-Phenylmethansulfonsäurerest ist.

18. Derivat nach Anspruch 1, dadurch gekennzeichnet, daß der Substituent in $N_2$-Stellung der (Trimethoxy-3,4,5)-Phenylmethansulfonsäurerest ist.

19. Derivat nach Anspruch 1, dadurch gekennzeichnet, daß der Substituent in $N_2$-Stellung der (Methylendioxy-3,4)-Phenylmethansulfonsäurerest ist.

20. Derivat nach Anspruch 1, dadurch gekennzeichnet, daß der Substituent in $N_2$-Stellung der Furyl-2-Methansulfonsäurerest ist oder dessen Natriumsalz.

21. Derivat nach Anspruch 1, dadurch gekennzeichnet, daß der Substituent in $N_2$-Stellung der Thienyl-2-Methan-sulfonsäurerest ist oder dessen Natriumsalz.

22. Derivat nach Anspruch 1, dadurch gekennzeichnet, daß der Substituent in $N_2$-Stellung der (Methoxy-2-hydroxy-4)-Methansulfonsäurerest ist.

23. Derivat nach Anspruch 1, dadurch gekennzeichnet, daß der Substituent in $N_2$-Stellung der Pyridyl-2-Methansulfonsäurerest ist oder dessen saure oder basische Salze.

24. Derivat nach Anspruch 1, dadurch gekennzeichnet, daß der Substituent in $N_2$-Stellung der Cyclohexylmethansulfonsäurerest ist oder dessen Natriumsalz.

25. Derivat nach Anspruch 1, dadurch gekennzeichnet, daß der Substituent in $N_2$-Stellung der (Äthoxy-3-hydroxy-4)-Phenylmethansulfonsäurerest ist oder dessen Natriumsalz oder dessen Salz mit einem Amin.

26. Derivat nach Anspruch 1, dadurch gekennzeichnet, daß der Substituent in $N_2$-Stellung der (Hydroxy-2-)-Phenylmethansulfonsäurerest ist oder dessen Natriumsalz oder dessen Salz mit einem Amin.

27. Verfahren zur Herstellung von Produkten gemäß Anspruch 1, dadurch gekennzeichnet, daß man in einem zweckentsprechenden Lösungsmittel wie Pyridin ein Derivat von Diamino-2,4-benzyl-5-pyrimidin mit einem Aldehyd und Schwefelsäureanhydrid umsetzt, worauf man das Reaktionsprodukt isoliert.

28. Verfahren nach Anspruch 27, dadurch gekennzeichnet, daß man stöchiometrische Mengen Diamino-2,4-benzyl-5-pyrimidin und Aldehyd einsetzt und daß man das Schwefelsäureanhydrid im Überschuß einsetzt.

29. Verfahren nach einem der Ansprüche 27 oder 28, dadurch gekennzeichnet, daß man das Reaktionsprodukt isoliert durch Verdünnung des Reaktionsgemisches mit einem mit Pyridin mischbaren Lösungsmittel wie Äther oder einem Kohlenwasserstoff.

30. Verfahren nach einem der Ansprüche 27 bis 29, dadurch gekennzeichnet, daß das isolierte Produkt durch Waschen mit wässrigem Alkohol gereinigt wird.

31. Medikament für die Humanmedizin und/oder Veterinärmedizin, dadurch gekennzeichnet, daß es mindestens eine Verbindung nach den Ansprüchen 1 bis 26 enthält und zwar in reinem Zustand oder in Verbindung mit einem oder mehreren anderen Wirkstoffen und einem oder mehreren pharmazeutisch akzeptablen Hilfsstoffen oder Verdünnungsstoffen.

**Claims**

1. Soluble N2 substituted derivatives of 2,4-diamino 5-benzyl pyrimidine responding to the following formula III:

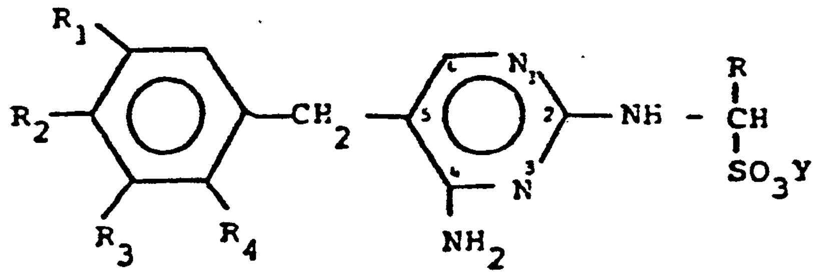

(III)

in which:

$R_1$, $R_2$, $R_3$, $R_4$ which may be identical or different, represent a hydrogen atom, a halogen atom or an alkyl, thioalkyl, alkoxybenzyloxy- or alkyloxyalkoxy- group,

Y represents a hydrogen atom, an alkali metal or a pharmaceutically compatible organic base, and

R represents a hydrogen atom or a linear or branched alkyl radical comprising from 1 to 7 carbon atoms, a cycloalkyl radical comprising from 5 to 8 carbon atoms, a phenyl radical possibly substituted by substituents such as, halogen, nitro-, hydroxyl, dialkylamino-alkoxyl-, alkyl from $C_1$ to $C_3$, two adjacent substituents being able to constitute an alkyldioxy-, furan, thiophene or pyridine ring.

2. Derivative according to Claim 1,characterized in that the substituent on the $N_2$ is the ethane sulfonic radical or its sodium salt.

3. Derivative according to Claims 1, characterized in that the substituent on the $N_2$ is the ethane sulfonic radical or its sodium salt.

4. Derivative according to Claim 1, characterized in that the substituent on the $N_2$, is the n-butane sulfonic radical.

5. Derivative according to Claim 1, characterized in that the substituent on the $N_2$, is the 2-methyl propane sulfonic radical.

6. Derivative according to Claim 1, characterized in that the substituent on the $N_2$, is the 2,2-dimethyl pentane sulfonic radical.

7. Derivative according to Claim 1, characterized in that the substituent on the $N_2$, is the 2,3-dimethyl pentane sulfonic radical.

8. Derivative according to Claim 1, characterized in that the substituent on the N2, is the 2-ethyl butane sulfonic radical.

9. Derivative according to Claim 1, characterized in that the substituent on the $N_2$, is the phenyl methane sulfonic radical.

10. Derivative according to Claim 1, characterized in that the substituent on the $N_2$, is the (2-chloro) phenyl methane sulfonic radical.

11. Derivative according to Claim 1, characterized in that the substituent on the $N_2$, is the (3-chloro) phenyl methane sulfonic radical.

12. Derivative according to Claim 1, characterized in that the substituent on the $N_2$, is the (4-chloro) phenyl methane sulfonic radical.

13. Derivative according to Claim 1, characterized in that the substituent on the $N_2$, is the (4-nitro) phenyl methane sulfonic radical.

14. Derivative according to Claim 1, characterized in that the substituent on the $N_2$, is the (3-nitro) phenyl methane sulfonic radical.

15. Derivative according to Claim 1, characterized in that the substituent on the $N_2$, is the (2-nitro) phenyl methane sulfonic radical.

16. Derivative according to Claim 1, characterized in that the substituent on the $N_2$, is the (2-methoxy) phenyl methane sulfonic radical.

17. Derivative according to Claim 1, characterized in that the substituent on the $N_2$, is the (3-methoxy) phenyl methane sulfonic radical.

18. Derivative according to Claim 1, characterized in that the substituent on the N2, is the (3,4,5-tri methoxy) phenyl methane sulfonic radical.

19. Derivative according to Claim 1, characterized in that the substituent on the $N_2$, is the (3,4-methylenedioxy) phenyl methane sulfonic radical.

20. Derivative according to Claim 1, characterized in that the substituent on the $N_2$, is the 2-furyl methane sulfonic radical or its sodium salt.

21. Derivative according to Claim 1, characterized in that the substituent on the N2, is the 2-thienyl methane sulfonic radical or its sodium salt.

22. Derivative according to Claim 1, characterized in that the substituent on the$N_2$, is the (2-methoxy 4-hydroxy) phenyl methane sulfonic radical.

23. Derivative according to Claim 1, characterized in that the substituent on the $N_2$, is the 2-pyridyl methane sulfonic radical or its acid or basic salts.

24. Derivative according to Claim 1, characterized in that the substituent on the $N_2$, is the cyclohexylmethane sulfonic radical or its sodium salt.

25. Derivative according to Claim 1, characterized in that the substituent on the $N_2$, is the (3-ethoxy 4-hydroxy) phenyl methane sulfonic radical, its sodium salt or its amine salts.

26. Derivative according to Claim 1, characterized in that the substituent on the N, is (2-hydroxy) phenyl methane sulfonic, its sodium salt or its amine salts.

27. Process for the preparation of the products according to Claim 1, characterized in that in a suitable solvent such as pyridine, particularly, a 2,4-diamino 5-benzyl pyrimidine is reacted, with an aldehyde and sulfurous anhydride, and then the product of the reaction is isolated.

28. Process according to Claim 2, characterized in that the amounts of 2,4-diamino 5-benzyl pyrimidine and aldehyde, are stoichiometric, whilst the sulfurous anhydride is used in excess.

29. Process according to any one of Claims 2 and 3, characterized in that the product of the reaction is isolated by dilution of the reaction medium by a solvent miscible with pyridine, such as ether or a hydrocarbon.

30. Process according to any one of Claims 2 to 4, characterized in that the product isolated is in addition purified by washing with aqueous alcohol.

31. Medicament useful in human and/or veterinary medicine, characterized in that it contains at least one compound according to Claims 1 to 26 in the pure state or in association with one or several other active principles and one or several compatible and pharmaceutically acceptable adjuvants or diluents.